# EUROPEAN PATENT APPLICATION

(11) **EP 4 386 376 A1**
(43) Date of publication of application: **19.06.2024**
(21) Application number: 22306923.8
(22) Date of filing: 16.12.2022
(51) Int. Cl.: G01N 33/00, G01N 33/04

(54) **VOLATILE COMPOUND DETECTION MODULE AND METHOD OF CONFIGURATION**

(71) Applicant: ALPHA M.O.S., 31500 Toulouse (FR)
(72) Inventor: BOYE, Julien, 31500 TOULOUSE (FR); MONROUSSEAU, Thomas, 31500 TOULOUSE (FR); PELLETIER, Sébastien, 31500 TOULOUSE (FR)
(74) Representative: Atout PI Laplace

(57) **Abstract**

To address the problem of measuring the quality of products in real time the system comprises a set of actuators and sensors capable of processing the gas to be measured without direct human intervention. This measurement may then be output for example by a wireless communication channel. The system is placed within a reservoir containing the analysis matrix and a sampling extractor enabling the circulation of the gas to be measured so as to bring it into contact with the gas sensors. The pump may be activated intermittently so as to regularly sample the air within the reservoir, leaving time between samples for the sensors to analyze the sample. The sensors used do not require any exterior intervention to operate, so that continuous operation is possible.

The assessment is periodic, in real time, and in situ. Sampling is performed by displacing air from the reservoir into an analysis chamber in which a network of sensors continuously measure Volatile Compound levels. The gaseous sample, once analyzed, may be reintroduced into the reservoir so as not to impoverish the matrix, particularly where contamination of the sample during the analysis can be excluded. Alternatively, the sample may be evacuated outside the reservoir after analysis.

## Description

### FIELD OF THE INVENTION

The invention relates to a system for monitoring the odor of a product in order to assess its quality or to detect a change.

### BACKGROUND OF THE INVENTION

Products (like dairy products) are generally stored in reservoirs for transportation from a collection point to a processing or packing location. These locations may be separated by thousands of kilometers and the reservoirs are typically transported by truck, boat or train to their final destination.

During transportation, product spoilage (like bacteria development) occurs in the reservoir at a greater or lesser rate depending on the initial quality of the product and the storage conditions in the reservoir. In particular, the reservoir may be cooled, or not, pressurized, or not, subject to a controlled atmosphere, and so on.

The increase in the number of bacteria is often correlated with an increase in Volatile Organic Compounds (Volatile Compound).

Known publications in this field include:
"Rapid methods and sensors for milk quality monitoring and spoilage detection" by Arshak Poghossian,Hanno Geissler and Michael J. Schoning in Biosensors and Bioelectronics 140 (2019) 111272;
"Application of E-nose technology combined with artificial neural network to predict total bacterial count in milk" by Yongheng Yang and Lijuan Wei in J. Dairy Sci. 104 https://doi.org/10.3168/jds.2020-19987;
"Characterization of Pasteurized Milk Spoilage by Electronic Nose in Relation to its Selected Quality Parameters by Saleem Ehsan, Zahir Al-Attabi, Nasser Al-Habsi, Michel R. G. Claereboud, and Mohammad Shafiur Rahman" in International Journal of Food Studies IJFS October 2021 Volume 10 pages 383-397;
"Analysis of pathogenic bacteria using exogenous volatile organic compound metabolites and optical sensor detection" by Emma Tait, Stephen P. Stanforth, Stephen Reed, John D. Perry and John R. Dean in RSC Adv., 2015, 5, 15494;
"Application of electronic nose and electronic tongue in the dairy industry" by M. TUDOR KALIT et al.: Mljekarstvo 64 (4), 228-244 (2014);
"Bacteria detection based on the evolution of enzyme-generated volatile organic compounds: Determination of Listeria monocytogenes in milk samples" by Emma Tait, John D. Perry, Stephen P. Stanforth, John R. Dean,in Analytica Chimica Acta 848 (2014) 80-87;
"Application of gas-sensor array technology for detection and monitoring of growth of spoilage bacteria in milk: A model study" by John Erik Haugen, Knut Rudi, Solveig Langsrud, Sylvia Bredholt Analytica Chimica Acta 565 (2006) 10-16;
"Identification of Volatile Organic Compounds Produced by Bacteria Using HS-SPME-GC-MS" by Emma Tait, John D. Perry, Stephen P. Stanforth and John R. Dean in the Journal of Chromatographic Science 2014;52:363-373; and
"Monitoring of rancidity of milk by means of an electronic nose and a dynamic PCA analysis" by Simonetta Capone in Sensors and Actuators B 78 (2001) 174-179*.*
"Shelf life determination by electronic nose: application to milk" by Said Labrecehg et al Sensors and Actuators 8 106 (2005) 199-206*.*

The analysis of the quality of dairy products is performed conventionally by bacteriological analysis, more specifically the SPC (Standard Plate Count) method. According to this method, the number of bacterial colonies developing under aerobic conditions as discussed for example in the article by Jayarao, B. M., S. R. Pillai, A. A. Sawant, D. R. Wolfgang, and N. V. Hegde. In 2004 entitled "Guidelines for monitoring bulk tank milk somatic cell and bacterial counts. J. Dairy Sci. 87:3561-73, and the "Grade A Pasteurized Milk Ordinance", 2007 Revision by U. S. Department of Health and Human Services, Public Health Service, Food and Drug Administration, is assessed. This measurement can be performed before the departure of a reservoir on a vehicle, on unloading from a vehicle, at release from customs, etc, but cannot be performed during transport. This technique requires physical access to the container, and the possibility of taking a sample, either or both of which may be practically or legally impossible.

In the article entitled by Said Labreche, Sandrine Bazzo, Sonia Cade, Eric Chanie. Dated 2005 entitled "Shelf life determination by electronic nose: application to milk. Sensors and Actuators" B 106, 199-206, in which the use of an electronic nose for the determination of the recommended use-by date of milk is presented.

Another article uses the Cyranose C320 electronic nose to detect the aging of pasteurized milk on the basis of Volatile Compounds.

Other articles suggest the use of electronic noses and electronic tongues in the Dairy industry for application such as the classification of samples, the determination of components responsible for the development of aromas, the microbial quality, the maturation of cheeses, the detection of mastitis, the detection of antibiotic residue or the detection of counterfeit milk.

Gas sensors are used in many applications, notably in situations where it is desired to detect or recognize a particular gas and in situations where it is desired to determine the composition of a gas mixture. In the present text, unless the context demands otherwise; the expression "gas" will be used to designate both a specific gas species and a mixture of different gaseous species, and the general expression "characterization" will be used to designate both the process of recognizing or detecting a particular gas and the process of determining the composition of a gas. It is to be understood that references in this text to a "gas sample" generally include references to any gas which is presented to the gas sensor (whether as a discrete sample or by exposing the sensor to an ambient gaseous medium).

Gas sensors have been developed using different sensing technologies, including for example chemoresistor type gas sensors, such as those based on semi-conducting metal-oxides.

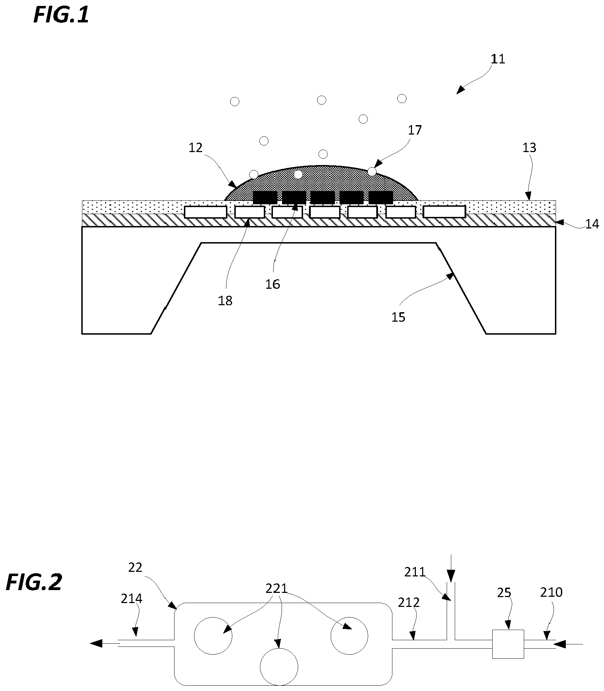

Figure 1 is a cross-sectional view which illustrates, schematically, the basic structure of a first prior art semi-conducting metal-oxide type gas sensor.

As shown in Figure 1, a semi-conducting metal-oxide type gas sensor 11 has a sensing layer made of semi-conducting metal-oxide region 12 provided on an insulating layer 13, over a membrane layer 14 supported on a base 15. When the sensor 11 is exposed to a gas, oxidation-reduction reactions (electron transfer) may occur between gas molecules 17 and the sensing layer 12, leading to a change in the impedance (conductance, capacitance, inductance or plural of these parameters) of the sensing layer 12. This change of impedance is measured using a pair of measuring electrodes 16 and heater 18 disposedbetween insulating layer 13 and membrane 14. Often the measurement is made by applying a bias voltage across the measurement electrodes and monitoring how the impedance presented by the sensing layer changes during gas exposure.

Figure 2 shows conventional sensor system. In order for an "electronic nose" device to assess and identify a wide range of different chemical signatures, it is usual to arrange a number of sensor devices such as those described with reference to figures 1, in an array. As shown in figure 2, there is provided a chamber 22, containing a plurality of sensors 221. The chamber 22 is connected via a conduit 212, 214. A sample for analysis is injected at injector port 211, where it mixes in a controlled manner with synthetic air provided at port 210, in flow meter 25. This synthetic air acts as a carrier, carrying the sample through each of the chambers, past each sensor, and finally out of an exhaust port 214. Typically, the sensors 221 will be of different types, or configured differently, so as to react differently to different sample compositions. By collating the response of each sensor to a given sample, the system can compile a "fingerprint" of the sample, which can then be compared to a library of known "fingerprints" to identify the closest match, and thereby the most plausible sample composition. The gas sensors may be configured (heating element temperature, measurement time, measurement cycle) so as to correspond to the measurement of the Volatile Compounds present, for example as described in EP3163295.

"Handbook of Machine Olfaction: Electronic Nose technology" by Tim C Pearce et al. edited by John Wiley & Sons, 24 January 2006 provides an introduction to the technical background in the field of the invention.

The articles "Catalytic combustion type hydrogen gas sensor using TiO2 and UV-LED" by Chi-Hwan Han et al published in Sensors and Actuators B 125 (2007) 224-228 and "Light enhanced gas sensing properties of indium oxide and tin dioxide sensors" by E. Comini et al published in Sensors and Actuators B 65 _2000. 260-263 may be consulted for further information concerning the state of the art.

Further discussion in this field may be found for example in the article by Beata Nalepa, Magdalena Anna Olszewska, Lidia Hanna Markiewicz: entitled "Seasonal variances in bacterial microbiota and volatile organic compounds in raw milk. International Journal of Food Microbiology 267 (2018) 70-76." or by Saleem Ehsan & al.: entitled "Characterization of Pasteurized Milk Spoilage by Electronic Nose in Relation to its Selected Quality Parameters" Published online 18 October 2021.

All of these prior art approaches are based on the preparation and analysis of samples in a laboratory, with an incubation of the samples at specified temperatures and an analysis using laboratory instruments.

These operations represent a high operating cost, laborious and time-consuming preparation steps, an extended analysis time and rely on trained and expert operators to interpret results.

The use of these instruments is limited to the specific application for which they are conceived, i.e., laboratory analysis.

It is desirable to enable autonomous testing, operable within a constrained space, in a vacuum or at high pressure, at temperatures close to 0°C.

### SUMMARY OF THE INVENTION

According to the present invention in a first aspect there is provided a detection module for incorporation in a hermetically closed container for a material susceptible to release volatile compounds. The module is configured to be mounted on the container in communication with a gaseous headspace therein. The detection module comprises a controller, a sample extractor, a sensor array, a gas permeable membrane, and a casing. The casing and the gas permeable membrane define together a closed chamber containing the sensor array and into which a portion of the contents of said gaseous headspace may be drawn through the gas-permeable membrane by operation of the sample extractor under the control of the controller while evacuating the previous contents of the chamber, whereby the outputs of said sensor array as exposed to said portion of the contents of said gaseous headspace are analyzed by said controller for the detection of volatile compounds of said material.

In accordance with a development of the first aspect, the material is a liquid.

In accordance with a development of the first aspect, the detection module further comprises a splash barrier configured to prevent a projection of said liquid into said closed chamber.

In accordance with a development of the first aspect, said volatile compounds are released from microorganisms.

In accordance with a development of the first aspect, the operation of the sample extractor under the control of the controller is performed while evacuating the previous contexts of the chamber back into said container.

In accordance with a development of the first aspect, the controller is further adapted to determine a concentration of a specified volatile compound, or rate of change therein, as constituting an emergency scenario, and performing an emergency operation.

In accordance with a development of the first aspect, the emergency operation comprises said operation of said sample extractor under the control of said controller while evacuating the previous contexts of said chamber to the outside of said chamber.

In accordance with a development of the first aspect, the chamber is further provided with a heating element controlled to maintain a temperature in the chamber such as to prevent condensation occurring therein.

In accordance with a development of the first aspect, the operation of said sample extractor under the control of said controller is performed so as to simultaneously evacuate the previous contexts of said chamber outside of said container.

In accordance with a development of the first aspect, the measurement or the emergency operation are accessed outside of the said container by way of wireless communication.

In accordance with a development of the first aspect, the controller is adapted to perform multivariate analysis-based machine learning to distinguish an emergency scenario.

In accordance with the invention in a second aspect there is provided a container comprising the module of the first aspect.

In accordance with the invention in a third as aspect there is provided a method for configuring a detection module as defined in the first aspect, the method comprising the steps of exposing said detection module to a first series of samples demonstrating a concentration of specified volatile compounds, or rate of change therein, as not constituting an emergency scenario, and exposing said detection module to a first series of samples demonstrating a concentration of specified volatile compounds, or rate of change therein, as constituting an emergency scenario, so as to train the multivariate analysis based machine learning capacity of said controller to distinguish an emergency scenario.

In accordance with a development of the third aspect the method comprises the further steps of copying a dataset representing the learned determination to a further detection module.

In accordance with the invention in a fourth as aspect there is provided a method of configuring a detection module as defined in the first aspect, the method comprising the steps of copying a dataset representing a learned determination thereto.

### BRIEF DESCRIPTION OF THE DRAWINGS

The above and other features, advantages and applications of the present invention will become more apparent from the following description of embodiments thereof, given by way of non-limiting examples, and the accompanying drawings, in which:
Figure 1 is a cross-sectional view which illustrates, schematically, the basic structure of a first prior art semi-conducting metal-oxide type gas sensor;
Figure 2 shows conventional sensor system;
Figure 3 presents a detection module in accordance with an embodiment;
Figure 4 shows results obtained from the monitoring of a orange juice reservoir using a system as described with respect to figure 3;
Figure 5 shows a detection module in accordance with a further embodiment;
Figure 6 shows a container in accordance with a further embodiment;
Figure 7 presents a method for configuring a detection module as discussed herein; and
Figure 8 shows a generic computing system suitable for implementation of embodiments of the invention.

### DETAILED DESCRIPTION OF CERTAIN EMBODIMENTS

In the case of a reservoir being transported e.g. by truck, boat or train it is unfeasible to extract a sample and to undertake a complex laboratory analysis.

A system adapted for this context is proposed which is autonomous, inexpensive and imposes no sample preparation steps. Embodiments support the measurement of Volatile Compounds present at the surface of a liquid contained in a hermetically closed reservoir.

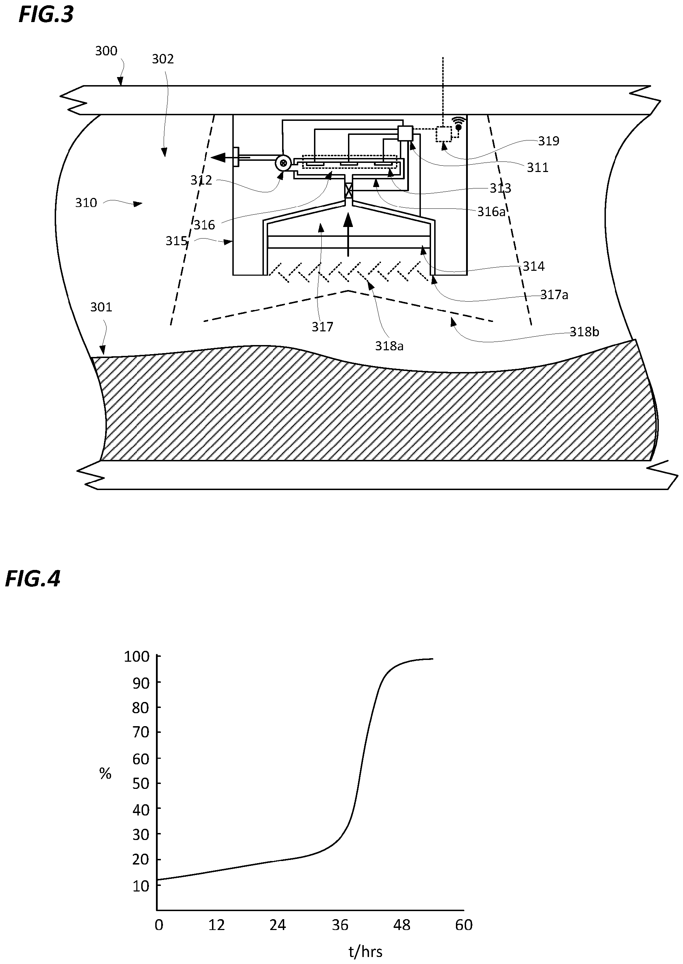

Figure 3 presents a detection module in accordance with an embodiment.

As shown, there is provided a detection module 310, for incorporation in a hermetically closed container 300 for a material 301 susceptible to release volatile compounds. Typical examples of such a material include milk, oils, fruit juices, cereals, seeds, farm effluent, human waste and so forth. The volatile compounds may for example be emitted by micro-organisms present in the matrix provided by the material. The material may be a liquid, solid or mixed phase material (sludge, slime...). The module 310 is configured to be mounted on the container 300 in communication with a gaseous headspace 302 therein. The detection module 310 comprises a controller 311, a sample extractor 312, a sensor array 313, a gas permeable membrane 314, and a casing 315. The casing 315 and said gas permeable membrane 314 define together a closed chamber 316 containing the sensor array 313. During operation, a portion of the contents of the gaseous headspace 302 is drawn through the gas-permeable membrane 314 by operation of the sample extractor 312 under the control of the controller 311 while evacuating the previous contents of the chamber 316. Thereby, the outputs of the sensor array as exposed to the portion of the contents of the gaseous headspace 302 are analyzed by the controller for the detection of volatile compounds of said material.

The controller 311 may comprise a microprocessor, microcontroller or other suitable data processing device.

The sample extractor 312 may comprise a pump, Diaphragm pump, piezo-electric pump, solenoid actuator, fan or the like. As such, embodiments provide a suction pump enabling the movement of a known volume from the headspace into a measurement chamber where gas sensors are provided.

The suction and circulation system are preferably sealed from liquid projections, and permeable to gas. A protection index of IP56 or IP57 may be appropriate for example. The mechanical construction of the system is preferable conceived with this in mind.

In the shown configuration, operation of the sample extractor under the control of the controller is performed while evacuating the previous contexts of said chamber back into the container, although in other embodiments the extraction of samples and evacuation may be performed as separate steps, e.g. with suitable valve or auxiliary pump arrangements. The evacuation may be performed to other destinations, for example to a waste port or waste reservoir outside the main reservoir, a sterilization unit within or outside the main reservoir, etc.

The sensor array 313 may comprise one or more sensors. These sensors may be of the same type, or comprise sensors of two or more different types, for example including a gas chromatograph, a gas sensor, a liquid sensor, a spectrometer, a biosensor, an ion specific electrode. Where similar sensors are provided in an array, the configuration of each may differ so that different components, albeit of similar type, may be provided to detect different Volatile Compounds, and/or be operational in the presence of different concentrations of Volatile Compounds.

The sensor array may be provided with additional heating elements, UV light sources or other ancillary devices as known in the art to improve or adjust the behaviour of each sensor.

Bacteria such as listeria are known to generate Volatile Compounds as discussed for example by Kalit M.T. Marković K. Kalit S. Vahćić N. Havranek J. in the article of 2015 entitled "Application of electronic nose and electronic tongue in the dairy industry. Mljekarstvo." 64: 228-244 , however the Volatile Compounds generated may be complex, and depend on the specific bacteria concerned, the season (see the article by EmmaTait, John D.Perry, Stephen P.Stanforth, John R.Dean: entitled "Bacteria detection based on the evolution of enzyme-generated volatile organic compounds: Determination of Listeria monocytogenes in milk samples." Analytica Chimica Acta 848 (2014) 80-87) and other factors. This makes it difficult in practice to determine the presence or absence of a specific bacterium on the basis of detected Volatile Compounds. On this basis certain embodiments propose a global sensor approach. That is to say, the sensor array measures a global VOC fingerprint, i.e. total VOC sensing approach to cover a large number of VOC released by a large number of bacteria (rather than focus on a particular bacteria that releases particular VOC).

The operation of each sensor, and/or the operating conditions thereof, may be controlled by the controller 311.

The gas permeable membrane 314 may be formed of a polymer such as PTFE, a stainless steel 316L mesh, polymer PE or nafion membrane. This membrane which is impermeable to liquid, but permeable to gas makes it possible ensure that only gas is captured.

The gas permeable membrane allows the passage of gas molecules to be analyzed while keeping liquid from the rest of the system. The membrane may also be removable for replacement e.g. when a degraded performance is detected.

A collection tunnel permits the channeling of gaseous samples to the measurement chamber.

Casing 315 may be formed of any suitable material, in view in particular of the requirement to achieve an air-tight container for the gas channel and sensors. The casing must also provide suitable chemical resistance to the contents of the reservoir 300, and the expected temperature range bearing in mind that certain embodiments call for heating elements to be housed within the casing, while on the other hand the contents of the reservoir 300 may be refrigerated to low temperatures. Suitable materials may include Stainless steel, aluminum alloys, Acrylonitrile Butadiene Styrene (ABS) High-Density Polyethylene (HDPE) Polyetheretherketone (PEEK), Acrylic (PMMA) Polypropylene (PP) and other materials as will readily occur to the skilled person. Preferably, the casing may be formed of food safe and/or biocompatible materials.

In certain embodiments, the module may be provided with one or more heating and/or cooling elements, configured to selectively control the temperature of parts of the module. For example, as shown the chamber 316 is provided with a heating element integrated in the wall 316a of the chamber. This heating element may then be controlled to maintain a temperature in the chamber, for example such as to prevent condensation occurring therein.

Similarly, as shown an optional funnel region 317 at the sample input, after the membrane 314, is provided with a heating element integrated in the wall 317a of the funnel region. This heating element may then be controlled to maintain a temperature in the funnel region, for example such as to prevent condensation occurring therein.

The processor 311 controls operation of the sensor array and receives the output thereof. The processor furthermore analyses the results to determine Volatile Compound levels.

The data gathered on the gas provides an indirect measurement of the level of contamination by micro-organisms. A statistical model may be used to transform the raw measurement from the gas sensors, providing an image of the contamination into a bacterial level or the state of the matrix depending on the programming of the system.

The assessment of the conservation quality of the matrix is performed on the basis of volatile compounds such as odor rather than counting bacterial colonies.

The system may use signal processing, statistical processing and machine learning such as described in EP3163295 and EP1566633 to detect any evolution, change or deviation in the measured Volatile Compounds, in connection with a bacteriological activity. This analysis may be performed out by means of multivariate analysis techniques such as k-NN (k-Nearest Neighbour), CA (Cluster Analysis), DFA (Discriminant Function Analysis), PCA (Principal Component Analysis), PCR (Principal Component Regression) Multiple Linear Regression (MLR), hierarchical cluster analysis (HCA) and the like. A problem of this approach is that these comparisons are not able to distinguish effectively between characteristic variations which are suggestive of a quality issue, and other, random sample variations which are of little interest. As such, these prior art techniques tend to either assess all samples as acceptable, or if very demanding criteria are set, assessing many analytes as not matching although the variations detected do not correspond to quality issues.

The analysis of samples may be performed on the basis of distinctions learned in a learning phase on the basis of training data as discussed below, or may be defined by prepared programming or configuration data, in which case there may be provided a method comprising the steps of copying a dataset representing a learned determination thereto.

As shown, the detection module may optionally further comprise an anti-immersion system 318b (dashed lines) and/or splash barrier 318a (dotted lines) configured to prevent a projection of said liquid into the closed chamber 316. Dotted and Dashed lines are used to associated elements of each system, however this does not necessarily indicate perforated materials. Either, both or neither structure 318a and/or 318b may be provided, or a hybrid structure comprising characteristics of both. 318b is an anti-immersion system to prevent liquid or solid entering in the headspace and allowing evacuation of the liquid/solid. 318a is an anti-splash barrier to prevent liquid/solid that may enter into the headspace to contaminate the membrane. This arrangement makes it possible to ensure that the measurement system always has access to the gas phase by preventing the projection of liquid on the filtration membrane within the reservoir. This may be configured as a function of the size and shape of the reservoir 300, the position of the detection module in the reservoir, the maximum fill level of the reservoir and the viscosity of the material 301.

An anti-immersion system and/or splash barrier, and/or anti-splash baffle as illustrated by 318a and/or 318b prevents the penetration of significant quantities of liquid and or solid particles into the measurement system. This baffle structure may be removable to facilitate cleaning.

An optional valve (including but not limited to one way valve, check valves) makes it possible to isolate the sensors from the rest of the system. A pump enables the circulation of the gaseous sample. The pump as shown is situated downstream of the measurement chamber so as to avoid possible contamination during the analysis of samples in the sensor chamber. The pump exhaust is directed either outside the reservoir, or filtered and channeled back into reservoir, so as to avoid any risk of contamination, bearing in mind that the internal sampling circuit may not be conveniently disinfected between samples.

As shown, the system may comprise an optional communications interface 319. This communications interface may allow access the controller 311 or other components for configuration, and/or for the retrieval of measurements. The interface may support communications via a hard wired electrical connection, and/or advantageously via a wireless interface. For example, the system may conveniently be interrogated at short-range protocol such a bluetooth, wifi, lorawan or long range (e.g. cellular communications), by any convenient protocol stack (such as over the world wide web) to retrieve the most recent measurement or any number of previous recorded measurements. This renders the current status of the analyzed matrix available more or less instantaneously, on demand.

The communications interface may optionally also be adapted to communicate with other local systems of the reservoir, for example to receive information concerning the reservoir status which might be relevant to the operations of the controller (vehicle navigation status (ETA...) reservoir volume, reservoir fill level, reservoir pressure, reservoir temperature, reservoir orientation, reservoir acceleration, reservoir location, etc). The communications interface may optionally be adapted to output information or instructions to other local systems, for example to a cooling system, navigation system, etc).

The system may optionally comprise a satellite based radio navigation receiver module such as a GPS receiver or the like in communication with the controller (not shown). This module may be used to determine the location of the container at any given time. This data may then be used to determine or derive weather (and in particular temperature and insolation values), atmospheric pressure, journey ETA information and so on, any of which may be used in sensor configuration and/or emergency condition determination.

The system parameters may be optimized to minimize electrical power consumption.

The system may minimize the impact of the heating from the electronic and fluid part of the system.

As such, the module preferably enables the measurement of Volatile Compounds without degrading the monitored product. Ambient air may be contaminated or heated by the measurement system. The system may be cleaned and/or disinfected between measurement cycles. The system may be provided with a cleaning system to this effect, capable for example of flushing the measurement channel with a neutral gas or fluid, solvent, disinfectant, or other cleaning medium as appropriate. The system may further comprise one or more ultraviolet light sources for disinfecting elements of the fluid circuit automatically. The system in view of its compact and self-contained structure may simply be swapped out with a replacement module as soon as a contamination is detected. Expelling exhaust air outside the reservoir limits the influence of the system, for example in the event of a contamination.

Figure 4 shows results obtained from the monitoring of an orange juice reservoir using a system as described with respect to figure 3.

Measured Volatile Compound levels are presented as a percentage of the measurement range of the sensor array on the y axis, and time in hours is presented on the x axis.

The curve represents measured Volatile Compound levels in an orange juice reservoir over a 60 hour period at ambient temperatures.

The increase over time of Volatile Compound levels is clearly visible, representing the degradation of the product due to growing microbial activity.

Specifically, as shown as an example, measurable Volatile Compound levels are detected when a first sample is aspirated by operation of the sample acquisition unit as discussed above, e.g. activation of a pump. At this stage a low Volatile Compound level of around 10 is measured. With successive measurements over time, the Volatile Compound level slowly increases to around 30 at 36 hours. From this point, Volatile Compound levels grow very rapidly, to saturate the sensor array after around 48 hours.

As such, embodiments make it possible to follow the evolution of a product's volatile compounds such as odor from a baseline in real time. The repeated automatic measurement of the evolution of Volatile Compound above the surface of the product (headspace) by means of a gas sensor makes it possible to follow this evolution over time.

On this basis, in certain embodiments the controller 311 may be further adapted to determine a concentration of specified volatile compound or a rate of change therein, as constituting an emergency scenario. This analysis may be based on a multivariate analysis based machine learning performed for example by the controller to distinguish an emergency scenario.

When such an emergency is determined, one or more emergency operations may be performed.

Where communications interface 319 is provided, emergency operations may comprise issuing a warning, e.g. to a driver, pilot or other crew member of the vehicle in charge of the vehicle transporting the reservoir, a central monitoring or control station, and so forth.

Where the reservoir is provided with a cooling system, emergency operations may comprise instructing the cooling system to lowering the temperature of the reservoir.

Where the reservoir is transported with a vehicle provided with a navigation system, emergency operations may comprise instructing the navigation system to select a faster route to destination.

Continuous measurement makes it possible to detect a microbial problem at the earliest juncture, and the sensitivity of the Volatile Compound sensors is preferably sufficient to assess compliance with relevant regulation. A pre-concentrator may be provided upstream of the sensors to increase the concentration of the Volatile Compounds to be measured.

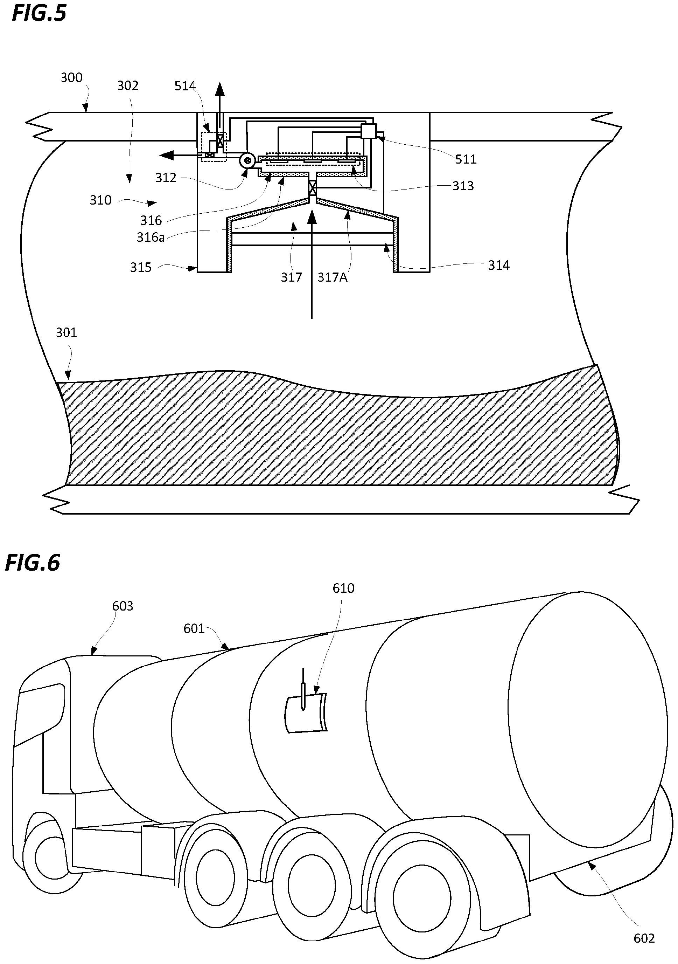

Figure 5 shows a detection module in accordance with a further embodiment.

As discussed above, different embodiments may encompass the expulsion of samples to a variety of destinations. In certain further embodiments, the module may be configured to selectively expel samples, by means for example of one or more valves. The embodiment of Figure 5 is identical to that of Figure 3, with the exception that a valve complex 514 is provided, configured to direct exhausted samples to two different destinations via respective exhaust ports. Specifically, as shown samples may be either exhausted to the exterior of the reservoir 300, or the interior. It will be appreciated that any number of such outputs may be envisaged, and that each output may be configured to exhaust samples to any of the destinations mentioned above, or otherwise. As shown, valves are provided so as to selectively direct a particular exhausted sample to one or the other exhaust ports. As shown, these valves may operate under the control of the controller 511, although alternative or additional control systems, including a manual selection may be envisaged.

As discussed with respect to figure 4, in certain situations an emergency operation may be performed. In the context of embodiments like that of figure 5, an example of an emergency operation may comprise operation of the sample extractor, for example under the control of said controller while evacuating the previous contexts of said chamber to a particular destination. For example, in certain embodiments while under ordinary operation samples may be exhausted back into the main reservoir, in the case of an emergency operation, corresponding for example to a detected bacteriological infection, the exhausted sample may be exhausted to the outside of said chamber.

Figure 6 shows a container in accordance with a further embodiment.

As shown in figure 6, there is provided a liquid container 601, as mentioned with respect to the foregoing embodiments. In particular, there is provided a cylindrical tank with a length along its main axis three to five times its diameter, and intended for storage and transport in a horizontal configuration. As such, this is a typical container for the long distance transportation of bulk liquids, of the type commonly used for the transportation of milk, oils, fruit juices, farm effluent, human waste and so forth. The container may be provided with cooling or heating systems, tracking systems and so on, as known to the skilled person.

As shown, the container 601 comprises a detection module 610, corresponding to the detection module as described herein.

As shown, the container 601 is mounted on a trailer 602 towed by a tractor 603.

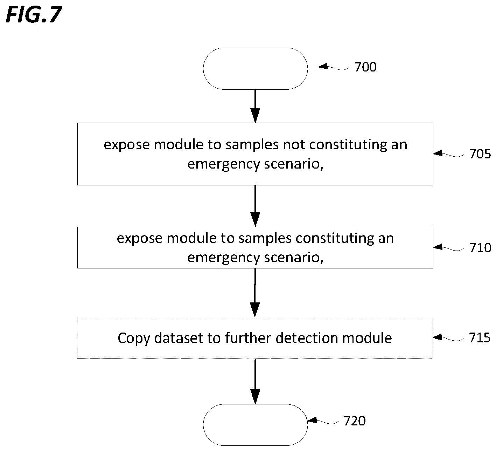

Figure 7 presents a method for configuring a detection module as discussed herein. As shown, the method starts at step 700, before proceeding to step 705 at which the detection module is exposed to a first series of samples demonstrating a concentration of specified volatile compounds, or rate of change therein, as not constituting an emergency scenario. As shown the method then proceeds to a step 710 of exposing the detection module to a first series of samples demonstrating a concentration of specified volatile compounds, or rate of change therein, as constituting an emergency scenario.

It will be appreciated that these two steps need not be performed in this order, and that they may in practice be performed in any order, or in parallel, or that both sets may be interspersed at random or in any other sequence.

By thus exposing examples of samples demonstrating an emergency scenario the multivariate analysis based machine learning capacity of said controller may be trained to distinguish an emergency scenario. On this basis, a module so trained will be enabled to operate in accordance with the embodiments presented for example with respect to figures 3, 5 or 6.

Optionally, as shown the method comprises further step 715 of copying a dataset representing said learned determination to a further detection module. On this basis, the trained behaviors learned in accordance with steps 705 and 710 may be propagated across multiple modules, so as to draw a maximum benefit from a single training operation.

The method may then terminate at step 720.

The disclosed methods can take form of an entirely hardware embodiment (e.g. FPGA), an entirely software embodiment (for example to control a system according to the invention) or an embodiment containing both hardware and software elements. Software embodiments include but are not limited to firmware, resident software, microcode, etc. The invention can take the form of a computer program product accessible from a computer-usable or computer-readable medium providing program code for use by or in connection with a computer or an instruction execution system. A computer-usable or computer-readable can be any apparatus that can contain, store, communicate, propagate, or transport the program for use by or in connection with the instruction execution system, apparatus, or device. The medium can be an electronic, magnetic, optical, electromagnetic, infrared, or semiconductor system (or apparatus or device) or a propagation medium.

These methods and processes may be implemented by means of computer-application programs or services, an application-programming interface (API), a library, and/or other computer-program product, or any combination of such entities.

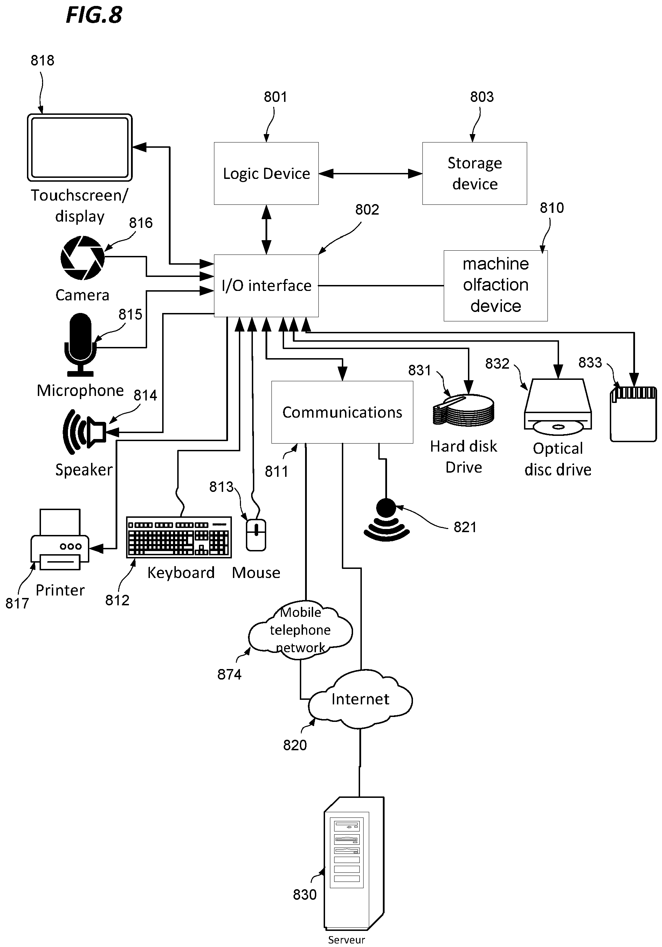

Figure 8 shows a generic computing system suitable for implementation of embodiments of the invention.

As shown in figure 8, a system includes a logic device 801, an I/O interface 802 and a storage device 803 connected to a machine olfaction device 810, corresponding for example to the sensor array 313 as presented in the embodiments of the invention. The system may optionally include a display subsystem 818, input subsystem 812, 813, 815, communication subsystem 811, and/or other components not shown.

Logic device 801 includes one or more physical devices configured to execute instructions. For example, the logic device 801 may be configured to execute instructions that are part of one or more applications, services, programs, routines, libraries, objects, components, data structures, or other logical constructs. Such instructions may be implemented to perform a task, implement a data type, transform the state of one or more components, achieve a technical effect, or otherwise arrive at a desired result.

The logic device 801 may include one or more processors configured to execute software instructions. Additionally or alternatively, the logic device may include one or more hardware or firmware logic devices configured to execute hardware or firmware instructions. Processors of the logic device may be single-core or multi-core, and the instructions executed thereon may be configured for sequential, parallel, and/or distributed processing. Individual components of the logic device 801 optionally may be distributed among two or more separate devices, which may be remotely located and/or configured for coordinated processing. Aspects of the logic device 801 may be virtualized and executed by remotely accessible, networked computing devices configured in a cloud-computing configuration.

Storage device 803 may include one or more physical devices configured to hold instructions executable by the logic device to implement the methods and processes described herein. When such methods and processes are implemented, the state of storage device 803 may be transformed-e.g., to hold different data.

Storage device 803 may include removable and/or built-in devices. Storage device 803 may comprise one or more types of storage device including optical memory (e.g., CD, DVD, HD-DVD, Blu-Ray Disc, etc.), semiconductor memory (e.g., RAM, EPROM, EEPROM, etc.), and/or magnetic memory (e.g., hard-disk drive, floppy-disk drive, tape drive, MRAM, etc.), among others. Storage device may include volatile, non-volatile, dynamic, static, read/write, read-only, random-access, sequential-access, location-addressable, file-addressable, and/or content-addressable devices.

In certain arrangements, the system may comprise an interface 802 adapted to support communications between the Logic device 801 and further system components, in particular the machine olfaction device 810. For example, additional system components may comprise removable and/or built-in extended storage devices. Extended storage devices may comprise one or more types of storage device including optical memory 832 (e.g., CD, DVD, HD-DVD, Blu-Ray Disc, etc.), semiconductor memory 833 (e.g., RAM, EPROM, EEPROM, FLASH etc.), and/or magnetic memory 831 (e.g., hard-disk drive, floppy-disk drive, tape drive, MRAM, etc.), among others. Such extended storage device may include volatile, non-volatile, dynamic, static, read/write, read-only, random-access, sequential-access, location-addressable, file-addressable, and/or content-addressable devices.

It will be appreciated that storage device includes one or more physical devices, and excludes propagating signals per se. However, aspects of the instructions described herein alternatively may be propagated by a communication medium (e.g., an electromagnetic signal, an optical signal, etc.), as opposed to being stored on a storage device.

Aspects of logic device 801 and storage device 803 may be integrated together into one or more hardware-logic components. Such hardware-logic components may include field-programmable gate arrays (FPGAs), program- and application-specific integrated circuits (PASIC/ASICs), program- and application-specific standard products (PSSP/ASSPs), system-on-a-chip (SOC), and complex programmable logic devices (CPLDs), for example.

The term "program" may be used to describe an aspect of computing system implemented to perform a particular function. In some cases, a program may be instantiated via logic device executing machine-readable instructions held by storage device. It will be understood that different modules may be instantiated from the same application, service, code block, object, library, routine, API, function, etc. Likewise, the same program may be instantiated by different applications, services, code blocks, objects, routines, APIs, functions, etc. The term "program" may encompass individual or groups of executable files, data files, libraries, drivers, scripts, database records, etc.

In particular, the system of figure 8 may be used to implement embodiments of the invention.

For example a program implementing the steps described with respect to figure 7 may be stored in storage device 803 and executed by logic device 801. The communications interface 811 may perform any of the functions ascribed to the communications module 319 above, and may in particular receive an error condition determination configuration or other configuration data from the server 830, and upload sample type information or sample characterization data as discussed above. The Logic device 801 may receive and compile the sample characterization, perform any additional processing, compare the final sample characterization with the characterization library, and indeed perform any or all of the functions ascribed to the controller 311 above, and optionally report the results to the user via communications module 811. At various stages of the operation further inputs, for example concerning the sample type, may be prompted via the display 818, and recovered via the user input interface devices 813, 812 as described above under the control of a suitable program, or may interface with internal or external dedicated systems adapted to perform some or all of these processes.

Accordingly the invention may be embodied in the form of a computer program.

It will be appreciated that a "service", as used herein, is an application program executable across multiple user sessions. A service may be available to one or more system components, programs, and/or other services. In some implementations, a service may run on one or more server-computing devices.

When included, display subsystem 818 may be used to present a visual representation of data held by storage device. This visual representation may take the form of a graphical user interface (GUI). As the herein described methods and processes change the data held by the storage device 803, and thus transform the state of the storage device 803, the state of display subsystem 818 may likewise be transformed to visually represent changes in the underlying data. Display subsystem 818 may include one or more display devices utilizing virtually any type of technology. Such display devices may be combined with logic device and/or storage device in a shared enclosure, or such display devices may be peripheral display devices.

When included, input subsystem may comprise or interface with one or more user-input devices such as a keyboard 812, mouse 813or game controller (not shown). In some embodiments, the input subsystem may comprise or interface with selected natural user input (NUI) componentry. Such componentry may be integrated or peripheral, and the transduction and/or processing of input actions may be handled on- or off-board. Example NUI componentry may include a microphone for speech and/or voice recognition; an infrared, colour, stereoscopic, and/or depth camera for machine vision and/or gesture recognition; a head tracker, eye tracker, accelerometer, and/or gyroscope for motion detection and/or intent recognition; as well as electric-field sensing componentry for assessing brain activity.

When included, communication subsystem 811 may be configured to communicatively couple computing system with one or more other computing devices. For example, communication module of may communicatively couple computing device to remote service hosted for example on a remote server 830 via a network of any size including for example a personal area network, local area network, wide area network, or the internet. Communication subsystem may include wired and/or wireless communication devices compatible with one or more different communication protocols. As non-limiting examples, the communication subsystem may be configured for communication via a wireless telephone network 874, or a wired or wireless local- or wide-area network. In some embodiments, the communication subsystem may allow computing system to send and/or receive messages to and/or from other devices via a network such as the Internet 820. The communications subsystem may additionally support short range inductive communications 821 with passive devices (NFC, RFID etc).

The system of figure 8 is intended to reflect a broad range of different types of information handling system. It will be appreciated that many of the subsystems and features described with respect to figure 8 are not required for implementation of the invention, but are included to more realistically reflect common systems. It will be appreciated that system architectures vary widely, and the relationship between the different sub-systems of figure 8 is merely schematic, and is likely to vary in terms of layout and the distribution of roles in real systems. It will be appreciated that in practice, systems are likely to incorporate different subsets of the various features and subsystems described with respect to figure 8.

Accordingly, to address the problem of measuring the quality of organic matter in real time embodiments may comprise a set of actuators and sensors capable of processing the gas to be measured without direct human intervention. This measurement may then be output for example by a wireless communication channel. The system may be placed within a reservoir containing the analysis matrix and a pumping system enabling the circulation of the gas to be measured so as to bring it into contact with the gas sensors. The pump may be activated intermittently so as to regularly sample the air within the reservoir, leaving time between samples for the sensors to analyze the sample. The sensors used do not require any exterior intervention to operate, so that continuous operation is possible.

The assessment may be periodic, in real time, and in situ. Sampling may be performed by displacing air from the reservoir into an analysis chamber in which a network of sensors continuously measure Volatile Compound levels. The gaseous sample, once analyzed, may be reintroduced into the reservoir so as not to impoverish the matrix, particularly where contamination of the sample during the analysis can be excluded. Alternatively, the sample may be evacuated outside the reservoir after analysis.

It will be understood that the configurations and/or approaches described herein are exemplary in nature, and that these specific embodiments or examples are not to be considered in a limiting sense, because numerous variations are possible. The specific routines or methods described herein may represent one or more of any number of processing strategies. As such, various acts illustrated and/or described may be performed in the sequence illustrated and/or described, in other sequences, in parallel, or omitted. Likewise, the order of the above-described processes may be changed.

The subject matter of the present disclosure includes all novel and non-obvious combinations and sub-combinations of the various processes, systems and configurations, and other features, functions, acts, and/or properties disclosed herein, as well as any and all equivalents thereof.

## Claims

1. A detection module for incorporation in a hermetically closed container for a material susceptible to release volatile compounds , said module configured to be mounted on said container in communication with a gaseous headspace therein, said detection module comprising a controller, a sample extractor, a sensor array, a gas permeable membrane, and a casing, said casing and said gas permeable membrane defining together a closed chamber containing said sensor array, and into which a portion of the contents of said gaseous headspace may be drawn through said gas-permeable membrane by operation of said sample extractor under the control of said controller while evacuating the previous contents of said chamber, whereby the outputs of said sensor array as exposed to said portion of the contents of said gaseous headspace are analyzed by said controller for the detection of volatile compounds of said material.

2. A module of claim 1 where material is a liquid.

3. A module of claim 2 where said detection module further comprises a splash barrier configured to prevent a projection of said liquid into said closed chamber.

4. A module of claim 1 where said volatile compounds are released from microorganisms present in said material.

5. The module of any preceding claim wherein said operation of said sample extractor under the control of said controller is performed while evacuating the previous contexts of said chamber back into said container.

6. The module of any preceding claim wherein said controller is further adapted to determine a concentration of a specified volatile compound, or rate of change therein, as constituting an emergency scenario, and performing an emergency operation.

7. The module of claim 6 wherein said emergency operation comprises said operation of said sample extractor under the control of said controller while evacuating the previous contexts of said chamber to the outside of said chamber.

8. The module of any preceding claim wherein said chamber is further provided with a heating element, said heating element being controlled to maintain a temperature in said chamber such as to prevent condensation occurring therein.

9. The module of any preceding claim wherein said operation of said sample extractor under the control of said controller is performed so as to simultaneously evacuate the previous contexts of said chamber outside of said container.

10. A module of claim 1 where the measurement or the emergency operation are accessed outside of the said container by way of wireless communication.

11. A module of any preceding claim wherein said controller is adapted to perform multivariate analysis based machine learning to distinguish an emergency scenario.

12. A container comprising the module of any preceding claim.

13. A method for configuring a detection module as defined in claim 12, said method comprising the steps of
exposing said detection module to a first series of samples demonstrating a concentration of specified volatile compounds, or rate of change therein, as not constituting an emergency scenario, and
exposing said detection module to a first series of samples demonstrating a concentration of specified volatile compounds, or rate of change therein, as constituting an emergency scenario,
so as to train the multivariate analysis based machine learning capacity of said controller to distinguish an emergency scenario.

14. The method of claim 13 comprising the further steps of copying a dataset representing said learned determination to a further said detection module.

15. A method of configuring a detection module as defined in any of claims 1 to 11 said method comprising the steps of copying a dataset representing a learned determination thereto.
